# EUROPEAN PATENT APPLICATION

(11) **EP 2 671 574 A1**
(43) Date of publication of application: **11.12.2013**
(21) Application number: 12170756.6
(22) Date of filing: 04.06.2012
(51) Int. Cl.: A61K 31/122, G01N 33/49, A61P 37/06

(54) **Use of vitamin K to decrease allograft failure and patient mortality after organ transplantation**

(71) Applicant: VitaK B.V., 6229 EV Maastricht (NL)
(72) Inventor: Vermeer, Cornelis, 6229 EV Maastricht (NL); De Borst, Martin Hendrik, 9741 CH Groningen (NL)
(74) Representative: Huygens, Arthur Victor

(57) **Abstract**

Circulating uncarboxylated matrix Gla-protein (ucMGP), in particular desphospho-ucMGP, is provided as a prognostic biomarker for allograft failure and mortality in patients who received organ transplantation and who are under immunosuppressive medication. A diagnostic tool is provided to monitor vitamin K status in such patients, i.e. the level of uncarboxylated species of extra-hepatic vitamin K-dependent proteins. Various forms and recommended dosages of vitamin K, optionally combined with vitamin D and/or other immunosuppressive medication, are provided to optimize the treatment of such patients resulting in decreased allograft failure and improved patient survival.

## Description

### Field of the Invention

The present invention is in the fields of clinical diagnostics and pharmacotherapy. In particular, the invention relates to the use of gamma-carboxyglutamatecontaining proteins as biomarkers for estimating the risk of allograft failure and mortality in transplant patients, and the use of vitamin K to reduce these risks.

### Background of the invention

Vitamin K may occur in two different forms: K1 and K2. Whereas K1 comprises one single chemical structure (phylloquinone), K2 is a group name for the family of menaquinones (abbreviated as MK-n), which have in common a methylated naphthoquinone ring structure as the functional group, but which vary in the length of their polyisoprenoid side chain. In the generally adopted nomenclature, n stands for the number of isoprenyl residues in MK-n. The number of isoprenyl residues in the side chain may vary from 1 (in MK-1) to 13 (in MK-13). The different forms of vitamin K share the function as coenzyme for the posttranslational enzyme gammaglutamate carboxylase (GCCX), but substantial differences have been reported with respect to absorption, transport, and pharmacokinetics {Schurgers LJ, Vermeer C. Biochim Biophys Acta 1570 (2002) 27-32*}*. Whereas K1 is preferentially utilized by the liver, K2 vitamins (mainly the long-chain menaquinones MK-7 through MK-10) are readily transported to extra-hepatic tissues, such as bone, arteries and adipose tissue. Commercially available K-vitamins include K1, MK-4 and MK-7.

The product of vitamin K action is the unusual aminoacid gammacarboxyglutamic acid, abbreviated as Gla. Presently, 17 Gla-containing proteins have been discovered and in those cases in which their functions are known they play key roles in regulating important physiological processes, including haemostasis, calcium metabolism, and cell growth and survival {Berkner KL, Runge KW. J Thromb Haemostas 2 (2004) 2118-2132}. Since new Gla-proteins are discovered almost every second year {Viegas CS et al. Am J Pathol 175 (2009) 2288-2298}, it is to be expected that more Gla-protein-controlled processes will be identified in the near future. In all Gla-proteins the function of which is known, the Gla-residues are essential for the activity and functionality of these proteins, whereas proteins lacking these residues are defective {Berkner KL, Runge KW. J Thromb Haemostas 2 (2004) 2118-2132*}.* The specificity with which Gla-domain structures facilitate interaction of vitamin K-dependent coagulation proteins with cell membranes is now becoming understood {Huang M et al. Nature Struct Biol 10 (2003) 751-756}*.* Likewise, it is well accepted that the Gla-residues of osteocalcin confer binding of the protein to the hydroxyapatite matrix of bone in a manner strongly suggestive of selectivity and functionality {Hoang QQ. Nature 425 (2003) 977-980}*.*

The Gla-proteins involved in haemostasis are all synthesized in the liver: four blood coagulation factors (II, VII, IX, and X) and three coagulation inhibiting proteins (C, S, and Z). In the normal healthy population, vitamin K intake is sufficient to cover the requirements of the liver, so in healthy adults all coagulation factors are fully carboxylated.

As will be detailed below, most extra-hepatic Gla-proteins are substantially under-carboxylated with 20-30% of the total antigen being present in the Gla-deficient (and hence inactive) state. Examples are the bone Gla-protein osteocalcin (OC) and the vascular Matrix Gla-Protein (MGP) {Knapen MH et al. Ann Int Med 111 (1989) 1001-1005*;* Cranenburg EC et al. Thromb Haemostas 104 (2010) 811-822}. Whereas the function of MGP as an inhibitor of soft tissue calcification is well understood {Schurgers LJ et al. Thromb Haem 100 (2008) 593-603*},* the function of OC has remained a matter of debate even 30 years after its discovery. The main function of the recently discovered Gla-rich protein (GRP) is probably also related to inhibiting calcification, notably in cartilage {Cancella ML et al. Adv Nutr 3 (2012) 174-181}*.*

Both carboxylated (cMGP) and uncarboxylated MGP (ucMGP) species have been detected in normal human plasma {Cranenburg EC et al. Thromb. Haemostas. 104 (2010) 811-822; Schurgers LJ et al. Blood 109 (2007) 3279-3283}. Whereas the total fraction of ucMGP (t-ucMGP) was found to be a "disease marker" that can be used for monitoring the amount of arterial calcium present {Cranenburg EC et al. Thromb. Haemostas. 101 (2009) 359-366}, the fraction known as desphospho-uncarboxylated MGP (dp-ucMGP) was shown to indicate overall vitamin K status and to be a "risk marker" directly associated with future calcification risk and cardiovascular mortality {Schurgers LJ et al. Clin. J. Am. Soc. Nephrol. 5 (2010) 568-575}*.*

Besides dp-ucMGP also conformation-specific assays for OC are commercially available [carboxylated OC (cOC) and uncarboxylated OC (ucOC)], respectively), and are often used to link vitamin K status with osteoporotic bone loss and fracture risk {Szulc PJ et al. J. Clin. Invest. 91 (1993) 1769-1774; Luukinen H et al. J. Bone Miner. Res. 15 (2000) 2473-2478}.

Conformation-specific assays for MGP have been described by Vermeer C, inter alia in the patent literature (EP 1190259, EP 1591791, US 6,746,847, US 7,700,296, and US 8,003,075). Their diagnostic use for cardiovascular disease and rheumatoid arthritis has been demonstrated. However, these patent publications are silent about the use of uncarboxylated MGP (notably dp-ucMGP) as a marker for immune response and organ transplant failure.

Recently, dietary vitamin K intake has been associated with inflammation. High vitamin K1 intake and high circulating vitamin K1 levels were found to be correlated with low levels of the inflammation marker CRP {Shea MK et al. Am J Epidemiol 167 (2008) 313-320}. The authors indicate that the mechanism may not be based on the classical function of vitamin K (posttranslational protein carboxylation) but on increased expression of inflammation-related genes. Inflammation may be the result of both the genetic makeup and an acquired immune response, and is typically accompanied by increased production of cytokines, including TNF-alpha, interleukin-1 and interleukin-6.

In rejection following organ transplantation, however, a prominent role has been attributed to different mechanisms, mainly those associated with an increase of HLA-proteins and T-lymphocytes {Sun HJ et al. Transpl. Immunol. 25 (2011) 42-48; Loupy A et al. Nat. Rev. Nephrol. 8 (2012) 348-357}. A role of vitamin K in this process has neither been described nor suggested so far.

The only disclosure of the use of vitamin K in transplantation patients is to treat vitamin K-deficiency related haemorrhages characterized by a prolonged prothrombin or partial thromboplastin time during the first postoperative week to avoid hemorrhagic complications (bleeding) {Prasad GV et al. Am J Kidney Dis 33 (1999) 963-965}-Obviously, the only purpose of this *short-term* application of vitamin K is to restore the hepatic vitamin K stores which may have been exhausted peri-operatively; in this way the blood coagulation system is normalized so that postoperative bleeding is prevented.

### Summary of the Invention

In a first aspect, the present invention provides the use of a pharmaceutical or nutraceutical composition comprising an effective amount of vitamin K for preventing or reducing allograft failure or patient mortality associated with allograft failure after organ transplantation in said patient.

Said use suitably is for the manufacture of a medicament or pharmaceutical or nutraceutical formulation comprising vitamin K for preventing or reducing allograft failure or patient mortality associated with allograft failure after organ transplantation in said patient.

Preferably, said vitamin K is vitamin K1 (phylloquinone) or vitamin K2 (menaquinone), in particular, in case of vitamin K2, MK-4 or one of the higher menaquinones MK-7, MK-8, MK-9 and MK-10.

Said pharmaceutical or nutraceutical composition is preferably administered to said patient over a period of at least 3 to 5 years, more preferably lifelong. Said patient usually is a mammal, in particular a human being.

The pharmaceutical or nutraceutical composition according to the invention is suitably and preferably administered in one of the following doses:
(a) when vitamin K is phylloquinone, between 5 and 10,000 micrograms per day, preferably between 50 and 5,000 micrograms per day, more preferably between 100 and 1,000 micrograms per day and most preferably between 200 and 500 micrograms per day;
(b) when vitamin K is menaquinone-4 (MK-4), between 5 and 15,000 micrograms per day, preferably between 50 and 5,000 micrograms per day, more preferably between 100 and 2,000 micrograms per day and most preferably between 200 and 1,000 micrograms per day;
(c) when vitamin K is any one of the long-chain menaquinones (MK-7 to MK-10), between 5 and 10,000 micrograms per day, preferably between 20 and 2,000 micrograms per day, more preferably between 50 and 1,000 micrograms per day and most preferably between 100 and 500 micrograms per day.

In a further aspect of the invention, the pharmaceutical or nutraceutical composition according to the invention is combined with an effective amount of vitamin D, preferably vitamin D3 (cholecalciferol). Said composition may also comprise vitamin D or, alternatively, vitamin D may be administered separately with the composition comprising vitamin K, together or in consecutive order.

In another aspect of the invention, the pharmaceutical or nutraceutical composition according to the invention is combined with one or immunosuppressive medicaments, including but not limited to cyclosporine, tacrolimus, mycophenolate mofetil, prednisone and azathioprine, or with a therapy for decreasing the risk of allograft failure in organ transplant patients. Such a therapy usually is a standard immune suppressive treatment which is known to a person skilled in the art..

In still a further aspect of the invention, a method is provided for estimating the risk of allograft failure and patient mortality associated with allograft failure after organ transplantation which comprises assessing circulating uncarboxylated and/or carboxylated forms of Gla-proteins as a biomarker in the blood of said patient.

Preferably, a conformation-specific assay is used for assessing one or more circulating uncarboxylated and/or carboxylated forms of Gla-proteins in the blood of said patient. Said one or more uncarboxylated and/or carboxylated forms of Gla-proteins are preferably selected from the group consisting of uncarboxylated and/or carboxylated species of osteocalcin (OC), uncarboxylated and/or carboxylated species of matrix Gla-protein (MGP), and uncarboxylated and/or carboxylated species of Gla-rich protein (GRP). Preferably, the circulating uncarboxylated forms of Gla-proteins are assessed, i.e. dp-ucMGP, ucOC and ucGRP, as this method is basically the more sensitive.

In a further aspect of the invention, the use of a conformation-specific assay is provided for either OC, MGP or GRP in the follow-up of patients after organ transplantation.

The organ transplantation to which the present invention primarily but not exclusively relates, is kidney transplantation and therefore the patients are primarily kidney transplant patients.

These and other aspects of the present invention will be more fully outlined in the detailed description which follows.

### Brief Description of the Drawings

- Figure 1:: Poor vitamin K status in patients after kidney transplantation. Error bars represent SD. The difference between patients with normal and low vitamin K intake was significant at p<0.04, the difference between the healthy reference group and patients with normal vitamin K intake was significant at p<0.005 (Student t-test).
- Figure 2:: Higher plasma dp-ucMGP is associated with increased risk of mortality. The three sub-groups represent tertiles (equal size) of the total patient population.
- Figure 3:: Higher plasma dp-ucMGP is associated with increased risk of cardiovascular mortality
- Figure 4:: Higher plasma dp-ucMGP is associated with increased risk of (non-censored) graft failure
- Figure 5:: Higher plasma dp-ucMGP is associated with an increased risk of the composite endpoint of mortality and graft failure
- Figure 6:: Significant fractions of both MGP and osteocalcin occur in their uncarboxylated (= vitamin K-deficient) conformation in healthy adults
- Figure 7:: Effect of high-dose of vitamin K1 (10 mg/day) on circulating dp-ucMGP levels
- Figure 8:: Effect of medium-dose of vitamin K1 (1 mg/day) on circulating dp-ucMGP levels
- Figure 9:: Effect of very high-dose of MK-4 (45 mg/day) on circulating dp-ucMGP levels
- Figure 10:: Effect of high-dose of MK-4 (15 mg/day) on circulating dp-ucMGP levels In Figures 7-10, error bars represent standard deviation (SD). The significance of differences (p-value) was calculated with the Student's t-test.

### Detailed Description of the Invention

The present invention relates in a first aspect to a new and completely different application of vitamin K, wherein vitamin K basically is to be administered *lifelong* to a patient to help prevent failure of a transplanted organ.

The invention is based on the surprising discovery that vitamin K status, as measured by the degree of carboxylation of circulating extrahepatic Gla-proteins, is inversely correlated with allograft failure and patient mortality in patients after kidney transplantation. Vitamin K status is regarded to be inadequate or insufficient when the circulating concentrations of uncarboxylated Gla-proteins exceed the upper normal value. For dp-ucMGP the upper normal value is set at 500 picomolar (pM).

Allograft failure, as used herein, is defined as acute or chronic loss of function of the transplanted organ and is considered an irreversible state of dysfunction of the transplanted organ. In the example of kidney transplantation, allograft failure is the loss of function so severe that the patient needs to return to renal replacement therapy (i.e. hemodialysis, peritoneal dialysis or re-transplantation). Usually this is required when GFR is <10 mL/min and patient has uremic complaints (encephalopathy, pericarditis etc), hyperkalemia, severe fluid retention etc.

As used herein, vitamin K status refers to the extent to which various Gla-proteins have been carboxylated. Poor carboxylation means that the vitamin K supply in the tissue from which the Gla-protein originates has been too low to ensure its full carboxylation. In general a distinction is made between hepatic vitamin K status (carboxylation of coagulation factors) and extra-hepatic vitamin K status. The liver produces the vitamin K-dependent blood coagulation factors. Insufficient hepatic vitamin K status is extremely rare; therefore, the clotting factors are no sensitive markers for vitamin K status. In this document a person's vitamin K status will be regarded as poor when the circulating level of the extra-hepatic protein MGP (measured as dp-ucMGP) is above the average normal level in healthy adults. Dp-ucMGP originates from other tissues than from the liver, mainly arteries and cartilage. Moreover, we provide evidence that also other extra-hepatic Gla-proteins may similarly be used as markers for extra-hepatic vitamin K status.

As used herein, study cohort is defined as the population (group of subjects, group of patients) in which the particular study has been performed.

For the purposes of this specification and appended claims, unless otherwise indicated, all numbers expressing quantities of ingredients, percentages or proportions of materials, reaction conditions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters set forth, the broad scope of the invention are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, e.g., 5.5 to 10.

It is noted that, as used in this specification and the appended claims, the singular forms "a," "an," and "the," include plural referents unless expressly and unequivocally limited to one referent. Thus, for example, reference to "a monomer" includes two or more monomers.

Reference will now be made in detail to certain embodiments of the invention, examples of which are illustrated in the accompanying drawings. While the invention will be described in conjunction with the illustrated embodiments, it will be understood that they are not intended to limit the invention to those embodiments. On the contrary, the invention is intended to cover all alternatives, modifications, and equivalents, which may be included within the invention as defined by the appended claims.

Preferably vitamin K and derivatives refer to one or more compounds of Formula 1', and/or their pharmaceutically or nutritionally acceptable salts, 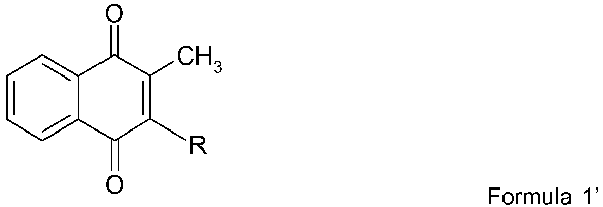 where R may be any covalently linked-organic group, including polyisoprenoid residues, esters, ethers, thiol adducts, etc. and especially the compounds thereof of Formula 2: 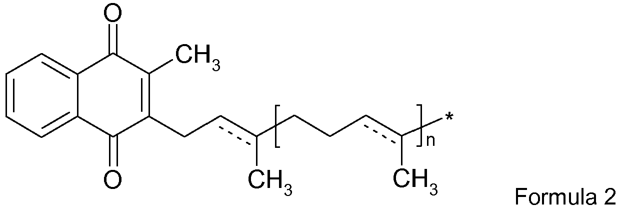 in which n is an integer from 1 to 12; and in which the broken lines indicate the optional presence of a double bond.

Vitamin K and derivatives thereof, as used herein, refer in particular to phylloquinone (also known as vitamin K₁), dihydrophylloquinone; menaquinone-4 (MK-4) and the long-chain menaquinones. It is generally accepted that the naphthoquinone is the functional group, so that the mechanism of action is similar for all K vitamins. Differences may be expected, however, with respect to intestinal absorption, transport, tissue distribution, and bioavailability. For use in the present invention, phylloquinone, MK-4 and long-chain menaquinones are preferred, and phylloquinone is particularly preferred.

Sources of vitamin K which can be used according to the present invention include the following: phylloquinone from natural sources, such as vegetable extracts, fats and oils, synthetic phylloquinone, synthetic vitamin K₃ (menadione), different forms of vitamin K₂: synthetic MK-4, MK-5, MK-6, MK-7, MK-8, MK-9, MK-10, MK-11, MK-12 and MK-13, natto (food prepared from fermented soy-bean, rich in MK-7), natto extracts, and other fermented foods or dairy products.

Vitamin K-enriched nutritional products can be manufactured to provide the daily requirements of vitamin K. For example, vitamin K can be added to food products, such as for example, meal replacers, ice cream, sauces, dressings, spreads, bars, sweets, snacks, cereals, beverages, etc. by methods as described in EP 1153548, the entire disclosure of which is incorporated by reference herein. Also, vitamin K can be used in food supplements such as multivitamins, tablets, capsules, sachets, and other forms.

The dose of vitamin K useful in performing the invention is not restricted but should be established per patient under guidance of the extent to which circulating dp-ucMGP or other uncarboxylated Gla-proteins are decreased as a result of the treatment. Current Al values or Adequate Intakes (as determined by the Institute of Medicine) are 120 µg for healthy men and 90 µg for healthy women. In patients, notably transplant patients, benefits may be derived by selecting dosages higher than the Al values. For example, suitable dosages may lie in the range 10 to 1,000 µg/day, more preferably 50 to 500 µg/day, and most preferably 100 to 200 µg vitamin K/day. Where national legislation permits, it may be advisable to provide dosage ranges as high as from 1 to 200 mg/day, preferably from 5 to 150 mg/day, and more preferably from 10 to 100 mg/day.

In terms of body weight, daily dosage may vary between 0.5 to 200 µg/kg body weight/day, preferably 0.75 to 25 µg/kg body weight/day, and more preferred 1 to 15 µg/kg body weight/day.

As used herein, the term "effective amount" or "therapeutically effective amount" are interchangeable and refer to an amount that results in bringing the plasma concentration of uncarboxylated Gla-proteins within the normal range, preferably around the lower-normal value. For example, an effective amount of vitamin K to normalize dp-ucMGP in renal transplant patients can be between about 50 µg/day and an upper limit of about 50 mg/day. In various embodiments, a dose between about 100 µg/day and about 2 mg/day is preferred. These doses are particularly useful in preventing allograft failure and reducing cardiovascular and overall mortality in patients who have received organ transplantation.

Vitamin D may be included together with vitamin K in the compositions described in this invention since it is well known that both osteocalcin and MGP have a vitamin D-responsive element in their promoter sequence and that expression of these proteins may thus be stimulated by vitamin D. Any form of natural or synthetic vitamin D may be employed, including vitamin D₁, vitamin D₂ (calciferol), vitamin D₃ (cholecalciferol) and vitamin D analogues (e.g. alfacalcidol, dihydrotachysterol, calcitriol). Natural sources of vitamin D, include saltwater fish, organ meats, fish-liver oils and egg yolk. Suitable dosages of vitamin D are 2 to 50 µg/day, preferably 5 to 20 µg/day, and most preferably about 7 to 10 µg/day.

The preferred treatment period starts shortly after organ transplantation and is continued lifelong. As there are no adverse side-effects associated with vitamin K supplementation, it should be regarded as an essential component for all patients who have experienced organ transplantation with the exception of those who also receive oral anticoagulant treatment on the basis of coumarin derivatives. Since coumarin derivatives act as vitamin K-antagonists, they cannot be used in combination with vitamin K.

The preferred route of administration of vitamin K is enterally, especially orally, but the parenteral or topical routes are viable alternatives. "Oral administration" as used herein includes oral, buccal, enteral or intragastric administration. The term "parenteral administration" as used herein includes any form of administration in which the vitamin K is absorbed into the blood stream without involving absorption via the intestines. Exemplary parenteral administrations that are used in the present invention include, but are not limited to intramuscular, intravenous, intraperitoneal, intraocular, subcutaneous or intra-articular administration.

Vitamin K is conventionally provided in the form of tablets or capsules, i.e. in a pharmaceutical or dietary supplement format. For pharmaceutical preparations or dietary supplements the vitamin K may be compounded with pharmaceutically acceptable carriers, excipients or diluents in the forms of pills, tablets (coated or uncoated), hard or soft capsules, dragées, lozenges, oral solutions, suspensions and dispersions, syrups or sterile parenteral preparations. Suitable excipients include inert diluents, such as calcium carbonate, sodium carbonate, lactose, calcium phosphate, sodium phosphate; granulating and disintegrating agents, such as cornstarch or alginic acid; binding agents, such as starch gelatin or acacia; effervescents; and lubricating agents, such as magnesium stearate, stearic acid or talc.

It is also possible to deliver or administer Vitamin K (optionally together with vitamin D) in a fortified food or beverage product. Preferred nutritional product formats include: juice drinks, dairy drinks, powdered drinks, sports drinks, mineral water, soy beverages, hot chocolate, malt drinks, biscuits, bread, crackers, confectioneries, chocolate, chewing-gum, margarines, spreads, yoghurts, breakfast cereals, snack bars, meal replacements, protein powders, desserts, and medical nutrition-tube feeds and nutritional supplements.

Conventional additives may be included in the compositions of the invention, including any of those selected from preservatives, chelating agents, effervescing agents, natural or artificial sweeteners, flavoring agents, coloring agents, taste masking agents, acidulants, emulsifiers, thickening agents, suspending agents, dispersing or wetting agents, antioxidants, and the like.

In various embodiments of the present invention, consumers are carriers of a transplanted organ, for instance a kidney; in those cases vitamin K (and optionally vitamin D) should be combined with other pharmaceutically active components currently used to limit or prevent rejection of the transplanted organ. For instance, it is envisaged that vitamin K could be provided in conjunction with medicaments selected from but not limited to: cyclosporin, tacrolimus, mycophenolate, mofetil, prednisone and azathioprine

In certain embodiments, the present invention includes the use of uncarboxylated or incompletely carboxylated species of MGP (here designated as dp-ucMGP) for monitoring the risk of transplant failure and mortality in patients following organ transplantation. The monitoring may start 1-2 months after surgery, when the patients have been stabilized. Subsequently, the monitoring may be repeated regularly, for instance once yearly.

In another embodiment, the present invention includes the use of other MGP species or total MGP antigen for monitoring the risk of transplant failure and mortality in transplant patients. These forms of MGP include carboxylated species (which will be higher when uncarboxylated species are lower) such as dp-cMGP, but also total uncarboxylated MGP (t-ucMGP) as a marker for arterial calcification and survival.

In another embodiment, the present invention includes the use of other conformation-specific assays, such as ucOC and cOC for monitoring the risk of transplant failure and mortality in transplant patients.

In still another embodiment, the present invention includes the use of various forms of vitamin K [notably phylloquinone (K1) and menaquinones (K2)] to improve survival of patients after organ transplantation surgery.

In still another embodiment, the present invention includes the use of various forms of vitamin K (notably phylloquinone and menaquinones) to decrease allograft failure in patients after organ transplantation surgery. The use of vitamin K for this purpose is recommended to be combined with other immunosuppressive drugs.

Having now generally described the invention, the same may be more readily understood through the following reference to the experimental part including the examples, which are provided by way of illustration and are not intended to limit the present invention unless specified.

### Experimental

The study cohort in which the present invention was made has been described in detail elsewhere {de Vries AP et al. Am. J Transpl. 4 (2004) 1675-1683}. In short, the Institutional Review Board of the Groningen University approved the study protocol (METC 2001/039), which was incorporated in the outpatient follow-up of the Groningen Renal Transplant Program. The outpatient follow-up constitutes a continuous surveillance system in which patients visit the outpatient clinic with declining frequency, in accordance with American Transplantation Society guidelines, i.e. ranging from twice a week immediately after hospital discharge to twice a year long-term after transplantation. Between August 2001 and July 2003, all adult allograft recipients who survived with a functioning allograft beyond the first year after transplantation (1 year post-transplant was considered *baseline)* were eligible to participate at their next visit to the outpatient clinic *(index date).* After the baseline visit, all participants visited the outpatient clinic at least once a year. Patients who had received a combined transplantation (i.e. kidney/pancreas or kidney/liver) were invited to participate as well. Patients with known or apparent systemic illnesses (e.g. malignancies or opportunistic infections) at index date were excluded from participation. A total of 606 out of 847 (72%) eligible renal transplant recipients signed written informed consent.

*Methods:* Relevant donor, recipient, and transplant characteristics were extracted from the Groningen Renal Transplant Database. This database holds information of all renal transplantations that have been performed at the center since 1968. In addition, the database contains the outcomes of outpatient visits (e.g. body weight, serum creatinine, creatinine clearance based on 24-h urine collection, and proteinuria) at 1 month, 6 months, 1 year, 2 years, and each following fifth year after transplantation. Extracted from the database were donor and recipient age, gender, ethnicity, primary renal disease, type and duration of dialysis therapy, type and date of transplantation, number of previous transplants, cold and warm ischemia times, number of human leukocyte antigen (HLA) mismatches, delayed graft function (i.e. days of post-transplant oliguria), cytomegalovirus (CMV) status, type of acute rejection treatment, body weight at baseline, 24-h creatinine clearance, and proteinuria at baseline. Smoking status at index date was obtained through a self-report questionnaire which had been sent to the participants via mail. Standard immunosuppression consisted of the following: Azathioprine (100 mg/d) and prednisolone from 1968 until 1989. Cyclosporin standard formulation (Sandimmune, Novartis: 10 mg/kg; trough-levels of 175-200 lg/L in the first 3 months, 150 lg/L between 3 and 12 months post-transplant, and 100 g/L thereafter) and prednisolone (starting with 20 mg/d, rapidly tapered to 10 mg/d) from January 1989 until February 1993. Cyclosporine microemulsion (Neoral, Novartis, Pharma b.v., Arnhem, the Netherlands; 10 mg/kg; trough-levels idem) and prednisolone from March 1993 until May 1996. Mycophenolate mofetil (Cellcept, Roche, Nederland b.v., Woerden, the Netherlands; 2 g/d) was added from May 1997 to date. Current medication was extracted from the medical record. Calcineurin inhibitor nephrotoxicity was defined as the discontinuation of calcineurin inhibitor use, or the conversion of one calcineurin inhibitor to the other between baseline and index date.

Blood was drawn after an 8-12-h overnight fasting period to prepare serum and citrated plasma, which were subsampled and frozen at -80 °C until use. Renal allograft function was assessed as the 24-h urinary creatinine clearance (CrCl), i.e. the 24-h urinary creatinine excretion divided by the serum creatinine concentration. Vitamin K status was assessed from plasma dp-ucMGP concentrations as described elsewhere {Cranenburg EC et al. Thromb. Haemostas. 104 (2010) 811-822}. The primary end-points of this study were death-censored graft failure and change in kidney function. Death-censored graft failure was defined as return to dialysis therapy or re-transplant. Change in kidney function was defined as estimated GFR (eGFR) at baseline subtracted by eGFR at the last follow-up visit, divided by time from baseline to follow-up and expressed in milliliters per minute per 1.73 m2 per year. This is preferred to a slope because kidney function decrease in chronic transplant dysfunction usually does not happen in a linear manner. Calculating a slope instead of a change in eGFR therefore will underestimate a patient's true kidney function decrease. For patients who died with a functioning graft (n=78), eGFR at follow-up was defined as kidney function at the last visit to the outpatient clinic before death, and for patients with graft failure (n=42), eGFR at follow-up was defined as the eGFR at the last visit to the outpatient clinic before starting dialysis therapy or re-transplant. There was no loss to follow-up.

Baseline plasma dp-ucMGP levels were measured by ELISA in a cohort of stable outpatient kidney transplant recipients (n=528) at 7.8 ± 6.4 (mean ± SD) years after transplantation. Associations between dp-ucMGP levels and the composite outcome of mortality and graft failure were analyzed by multivariate Cox regression analysis adjusting for age, gender, eGFR, proteinuria, hemoglobin, waist circumference, smoking, diabetes, total cholesterol, HDL cholesterol and systolic blood pressure. Sensitivity analyses were performed in a subgroup with eGFR >30 and <90 ml/min.

*Results:* Median plasma dp-ucMGP levels amounted 1039 pmol/L and 91% of the population was above the upper-normal level of 500 pmol/L indicating vitamin K insufficiency. After 6.4 ± 1.7 years of follow-up beyond baseline, 171 (28%) died or developed graft failure. Plasma dp-ucMGP levels were positively associated with an increased risk of the combined endpoint of all-cause mortality and graft failure (full model hazard ratio 1.86 per 1 SD increase [95% Cl 1.23-2.82], p=0.004). Analyses per tertile of dp-ucMGP and sensitivity analyses provided similar results.

*Conclusions:* Plasma dp-ucMGP is an independent risk factor for mortality and graft failure after kidney transplantation. Increased vitamin K intake, resulting in decreased dp-ucMGP levels, is a logical strategy to decrease allograft failure and mortality in patients following organ transplantation.

### Example 1

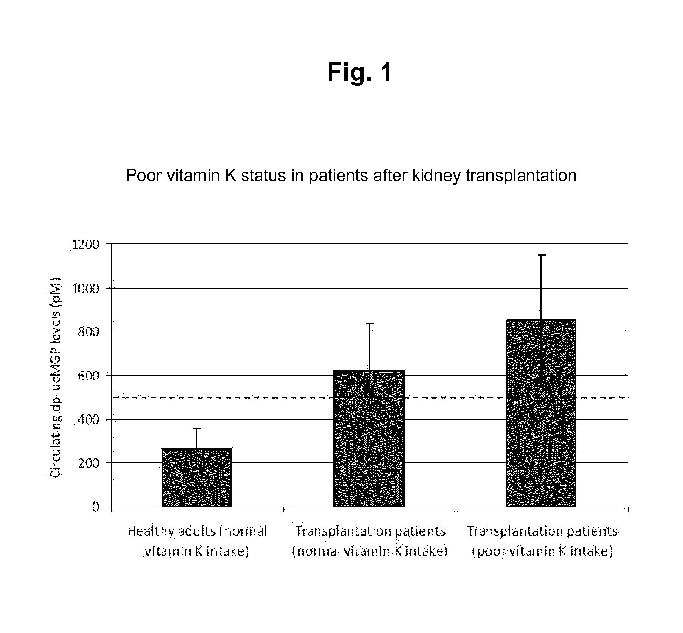

Transplantation patients have a poor vitamin K status as decided from their plasma dp-ucMGP levels (Figure 1). In 60 kidney transplant recipients, vitamin K intake was estimated using four-day food records. Total vitamin K intake was below the recommended level (men: 120 µg/day, women: 90 µg/day) in 50% of all subjects, both in men (15/30 patients) and women (15/30 patients). Vascular vitamin K status was assessed by measuring circulating dp-ucMGP and compared with 60 age- and sex-matched healthy subjects with adequate vitamin K intake. The majority of all patients had plasma dp-ucMGP levels above the normal range, which is indicative for poor vascular vitamin K status. Patients with vitamin K intakes below the recommended intake had higher dp-ucMGP levels than those with adequate intakes.

### Example 2

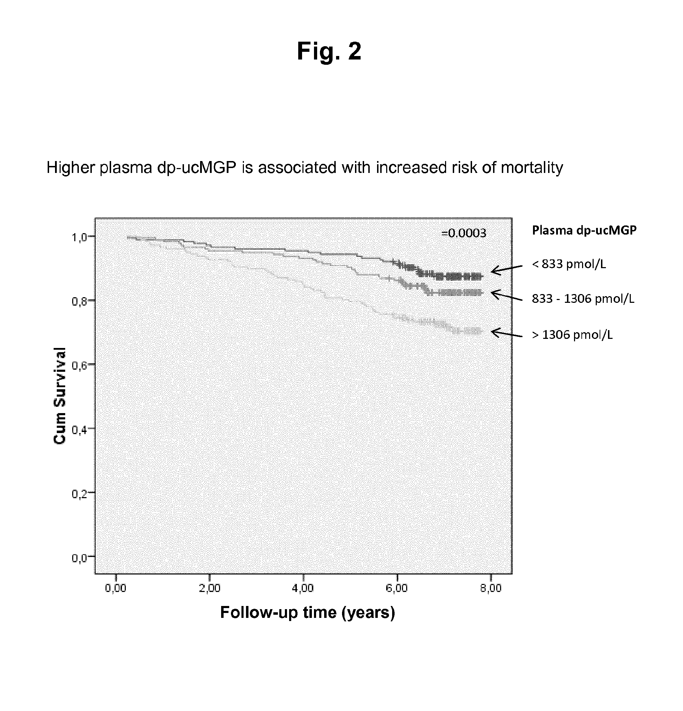

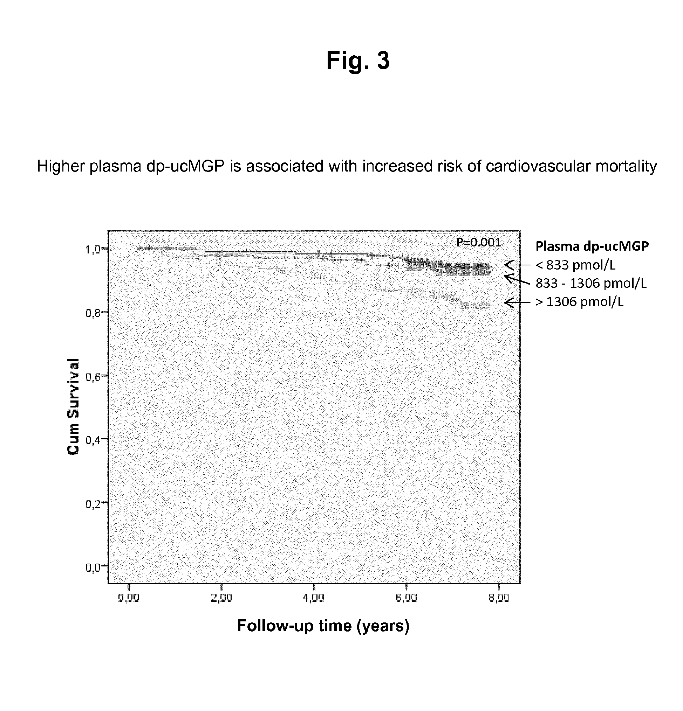

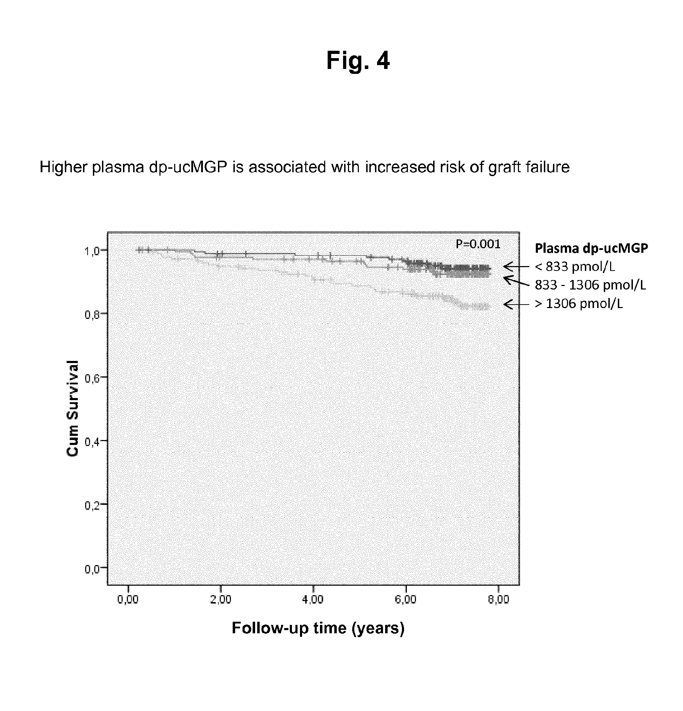

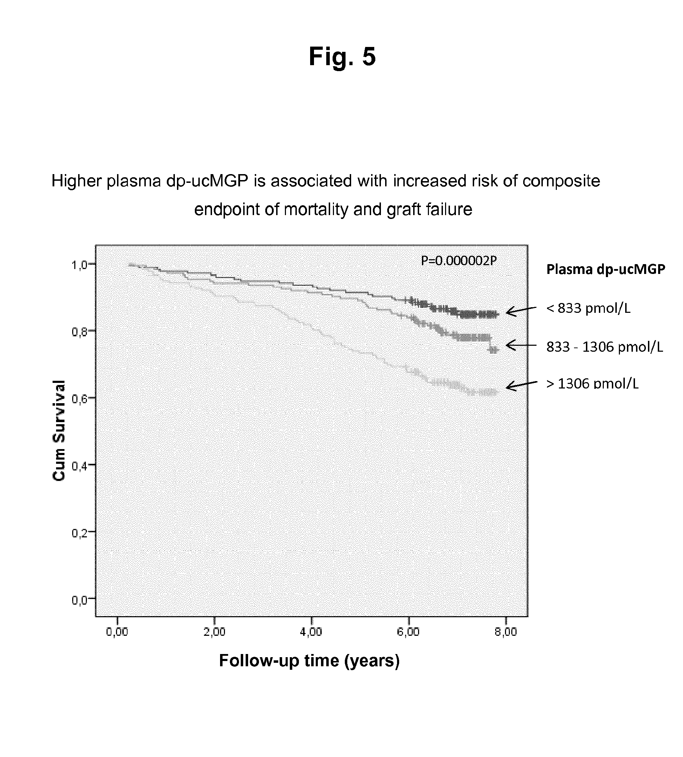

Plasma dp-ucMGP is a marker for allograft failure and transplant recipient mortality (Figures 2-5). In the study cohort, 91% of transplant patients had dp-ucMGP levels >500 pmol/L (upper level of the normal range in healthy adults). The median [IQR] in the present population (skewed) was 1039 [733-1542] pmol/L. The total study cohort (n=528) was divided into tertiles and in each tertile patients were monitored over 7 years after transplantation. In Figures 2-5 the cumulative all-cause mortality, cardiovascular mortality, allograft failure and the composite all-cause mortality + graft failure are shown. Table 1 below shows the detailed statistics for each of these figures.

**Table 1**

| **Cox regression analysis for** **Figures 2-5** | | | |
|---|---|---|---|
| **dp-ucMGP levels (per 1 µmol/L) and outcomes** | | | |
| **Crude** | | | |
| | st. beta | 95%Cl | P |
| mortality | 1.36 | 1.19-1.55 | <0.001 |
| CV mortality | 1.43 | 1.20-1.70 | <0.001 |
| graft failure | 1.49 | 1.23-1.81 | <0.001 |
| composite | 1.39 | 1.24-1.56 | <0.001 |

| **Age & gender adjusted** | | | |
|---|---|---|---|
| | st. beta | 95%Cl | P |
| mortality | 1.28 | 1.11-1.48 | 0.001 |
| CV mortality | 1.34 | 1.11-1.61 | 0.001 |
| graft failure | 1.56 | 1.28-1.89 | <0.001 |
| composite | 1.33 | 1.18-1.50 | <0.001 |

| **Adjusted for age, gender, creatinine clearance, proteinuria** | | | |
|---|---|---|---|
| | st. beta | 95%Cl | P |
| mortality | 1.23 | 1.05-1.45 | 0.012 |
| CV mortality | 1.31 | 1.06-1.61 | 0.011 |
| graft failure | 1.29 | 1.01-1.65 | 0.04 |
| composite | 1.26 | 1.09-1.44 | 0.001 |

| **Adjusted for age, gender, creatinine clearance, proteinuria, Hb, use of ACEi/ARB, waist circumference, smoking, diabetes, total cholesterol, HDL cholesterol and systolic blood pressure** | | | |
|---|---|---|---|
| | st. beta | 95%Cl | P |
| mortality | 1.20 | 1.02-1.41 | 0.02 |
| CV mortality | 1.30 | 1.07-1.60 | 0.01 |
| graft failure | 1.35 | 1.00-1.82 | 0.048 |
| composite | 1.22 | 1.06-1.41 | 0.005 |

### Example 3

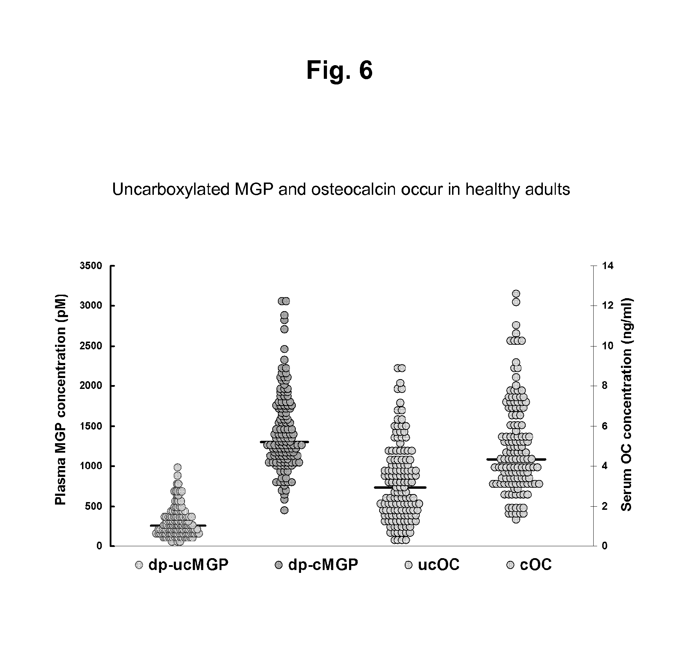

Both dp-ucMGP and ucOC are found in the circulation in healthy elderly subjects (55-65 years of age) (Figure 6). Both carboxylated and uncarboxylated species of osteocalcin and MGP were measured using conformation-specific assays as described elsewhere. It resulted that between 20 and 30% of the circulating antigens occurred in the uncarboxylated state. The four different tests shown in this figure were performed in samples from the same individuals. A significant correlation (R² = 0.5) was found between ucOC and dp-ucMGP. This demonstrates that both dp-ucMGP and ucOC are markers that can be used to detect poor vitamin K status. A normal range for dp-ucMGP was defined between 200 and 500 pM.

### Example 4

Recommended doses of vitamin K for treating transplant patients. In healthy subjects, the response to vitamin K supplements shows large inter-individual variation. On top of this, chronic kidney disease (CKD) patients are known to have much more pronounced vitamin K insufficiency than healthy subjects {Schlieper G et al. J. Am. Soc. Nephrol., 22 (2011) 387-395*]*. Therefore, the estimated range for optimal effect is dependent on the disease state and may be higher than estimated from literature data. Below we will substantiate the recommended doses of vitamin K for treating transplant patients.

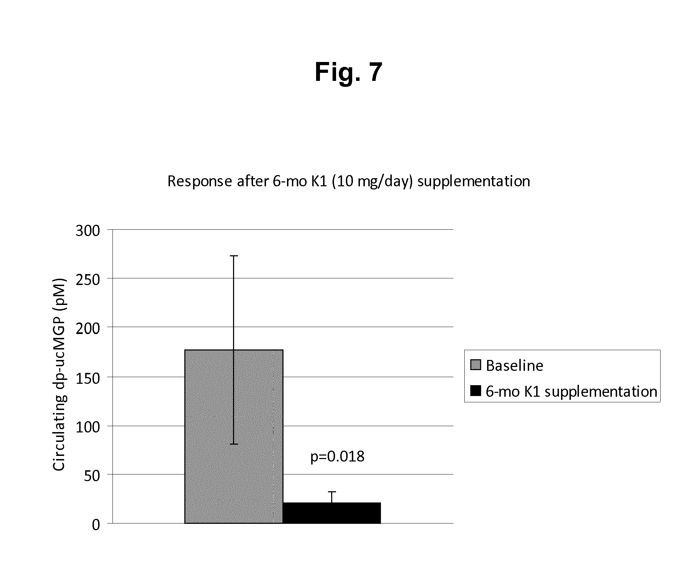

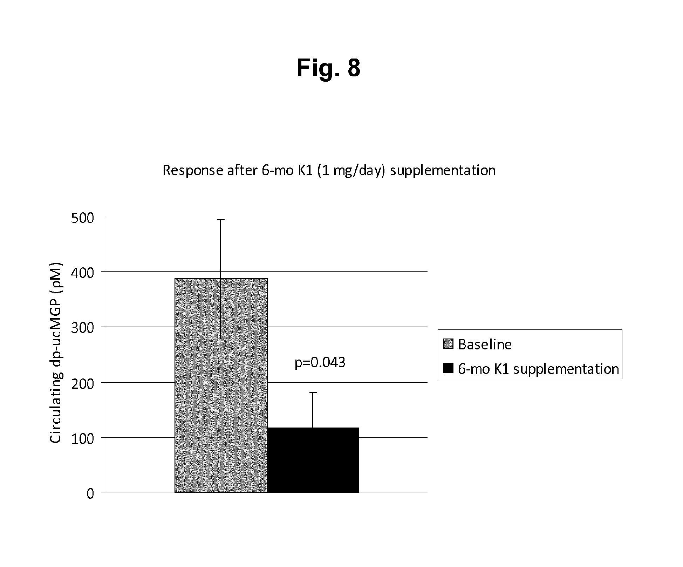

K1: **10,000 µg/day** resulted in more than 95% carboxylation of circulating OC and MGP and a concomitant decrease of the corresponding uncarboxylated species (ucOC and dp-ucMGP, respectively). This was shown in archived samples from a vitamin K1-intervention study initially described in 2003 {Braam LA et al. Am. J. Sports Med. 31 (2003) 889-895}. In the vitamin K1-treated group, circulating ucOC levels dropped during the first 6 months of the 2-year study from 4.6 ng/mL to levels that were not different from background (=buffer) values. The decrease in circulating dp-ucMGP is shown in Figure 7. **1****,000 µg/day** resulted in a substantial increase of OC and MGP carboxylation in healthy volunteers. This was shown in archived samples from a vitamin K1-intervention study initially described in 2003 {Braam LA et al. Calcif. Tissue Int. 73 (2003) 21-26}. During the first 6 months of the study, circulating ucOC in the vitamin K-treated group decreased from 4.2 ng/mL to 0.8 ng/mL, whereas in the control samples a non-significant increase was observed from 4.4 ng/mL to 4.7 ng/mL. The decrease in circulating dp-ucMGP is shown in Figure 8. **140 µg/day** showed a minor but statistically significant effect on the carboxylation degree of circulating OC {Schurgers LJ et al. Blood. 109 (2007) 3279-3283}.

Conclusion: the optimal dose for Gla-protein carboxylation in subjects free from CKD lies between 5 and 10,000 µg/day, preferably between 50 and 5,000 µg/day, more preferably between 100 and 1,000 µg/day and most preferably between 200 and 500 µg/day for subjects with baseline levels in the normal range. For some groups of patients, notably kidney patients with more pronounced vitamin K insufficiency, the required dosages may be 2-5 fold higher. Treatment should be guided by the circulating levels of dp-ucMGP or uncarboxylated species of any other suitable Gla-protein.

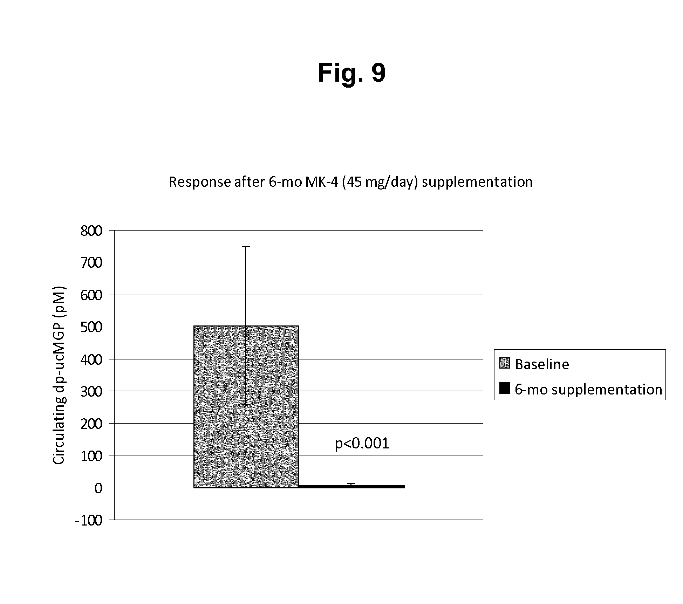

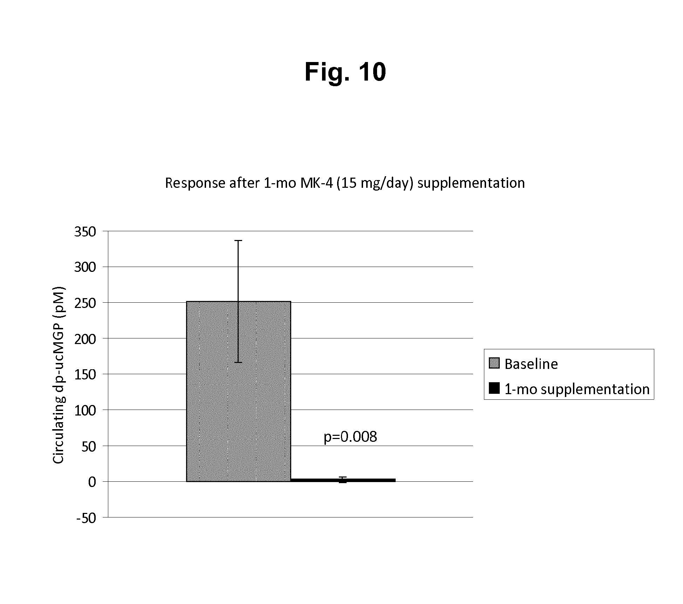

MK-4: The circulating half-life time of MK-4 is 1 h, that of vitamin K1 is around 1.5 h {Schurgers, L.J et al. Haemostasis 30 (2000) 298-307}*.* Therefore, slightly higher doses of MK-4 should be used than of vitamin K1 to obtain an optimal result. **45,000 µg/day** taken for 6 months resulted in complete carboxylation of circulating OC {Knapen, M.H.J. et al. Osteoporosis International 18 (2007) 963-972}. The decrease in circulating dp-ucMGP is shown in Figure 9.
**15,000 µg/day** during 1 month was used in non-published studies and similar results were obtained as for 45 mg/day. The decrease in circulating dp-ucMGP is shown in Figure 10.
**Lower doses** were tested in a number of small studies with no clear effect of 200 µg/day and a significant effect at 500 µg/day.

Conclusion: the optimal dose for Gla-protein carboxylation in subjects free from kidney disease lies between 5 and 15,000 µg/day, preferably between 50 and 10,000 µg/day, more preferably between 100 and 2,000 µg/day and most preferably between 200 and 1,000 µg/day for subjects with baseline levels in the normal range. For some groups of patients, notably kidney patients with more pronounced vitamin K insufficiency, the required dosages may be 2-5 fold higher. Treatment should be guided by the circulating levels of dp-ucMGP or uncarboxylated species of any other suitable Gla-protein.

MK-n: The long-chain menaquinones are selected from the group menaquinone-7, menaquinone-8, menaquinone-9, and menaquinone-10. The long half-lifes of the long-chain menaquinones make that the optimal dose for Gla-protein carboxylation is lower than for vitamin K1 {Schurgers LJ et al. Biochim. Biophys. Acta 1570 (2002) 27-32}. Their efficacy in terms of Gla-protein carboxylation is at least 2-3 fold higher, probably up to 6-fold higher than that of either K1 or MK-4 {Schurgers LJ et al. Blood 109 (2007) 3279-3283}.
**90 µg/day** {Theuwissen, E. et al. Br J Nutr 2012 PMID: 22289649} and **140 µg/day** {Schurgers, L.J. et al Blood 109 (2007) 3279-3283} were shown to have a substantial and statistically significant effect on OC and MGP carboxylation, whereas higher dosages were used in patient studies with CKD. A clear trend of improved MGP carboxylation was found, for instance, between **45 µg/day** and **360 µg/day** {Westenfeld R et al. Am. J. Kidney Dis. 59 (2012) 186-195}.

Conclusion: the optimal dose for Gla-protein carboxylation in subjects free from CKD lies between 5 and 5,000 µg/day, preferably between 25 and 2,000 µg/day, more preferably between 50 and 1,000 µg/day and most preferably between 100 and 500 µg/day for subjects with baseline levels in the normal range. For some groups of patients, notably kidney patients with more pronounced vitamin K insufficiency, the required dosages may be 2-5 fold higher. Treatment should be guided by the circulating levels of dp-ucMGP or uncarboxylated species of any other suitable Gla-protein.

## Claims

1. Use of a pharmaceutical or nutraceutical composition comprising an effective amount of vitamin K for preventing or reducing allograft failure or patient mortality associated with allograft failure after organ transplantation in said patient.

2. Use of a pharmaceutical or nutraceutical composition according to claim 1, wherein said vitamin K is vitamin K1 (phylloquinone) or vitamin K2 (menaquinone), in particular MK-4 or one of the higher menaquinones MK-7, MK-8, MK-9 and MK-10.

3. Use of a pharmaceutical or nutraceutical composition according to claim 1 or 2, wherein said pharmaceutical or nutraceutical composition is administered to said patient over a period of at least 3 to 5 years, preferably lifelong.

4. Use of a pharmaceutical or nutraceutical composition according to claim 3, wherein said patient is a mammal, in particular a human being.

5. Use of a pharmaceutical or nutraceutical composition according to any one of claims 1 to 4, wherein vitamin K is administered in the following dose:
(a) when vitamin K is phylloquinone, between 5 and 10,000 micrograms per day, preferably between 50 and 5,000 micrograms per day, more preferably between 100 and 1,000 micrograms per day and most preferably between 200 and 500 micrograms per day;
(b) when vitamin K is menaquinone-4 (MK-4), between 5 and 15,000 micrograms per day, preferably between 50 and 5,000 micrograms per day, more preferably between 100 and 2,000 micrograms per day and most preferably between 200 and 1,000 micrograms per day;
(c) when vitamin K is any one of the long-chain menaquinones (MK-7 to MK-10), between 5 and 10,000 micrograms per day, preferably between 20 and 2,000 micrograms per day, more preferably between 50 and 1,000 micrograms per day and most preferably between 100 and 500 micrograms per day.

6. Use of a pharmaceutical or nutraceutical composition according to any one of claims 1 to 5, wherein said pharmaceutical or nutraceutical composition is combined with an effective amount of vitamin D, preferably vitamin D3 (cholecalciferol).

7. Use of a pharmaceutical or nutraceutical composition according to claim 6, wherein said pharmaceutical or nutraceutical composition is administered together or in consecutive order with said effective amount of vitamin D.

8. Use of vitamin K according to any one of claims 1 to 7, wherein wherein said pharmaceutical or nutraceutical composition is combined with one or immunosuppressive medicaments or with a therapy for decreasing the risk of allograft failure in organ transplant patients.

9. A method for estimating the risk of allograft failure and patient mortality associated with allograft failure after organ transplantation which comprises assessing circulating uncarboxylated and/or carboxylated forms of Gla-proteins as a biomarker in the blood of said patient.

10. A method according to claim 9, wherein a conformation-specific assay is used for assessing one or more circulating uncarboxylated and/or carboxylated forms of Gla-proteins in the blood of said patient.

11. A method according to claim 10, wherein said one or more uncarboxylated and/or carboxylated forms of Gla-proteins are selected from the group consisting of uncarboxylated and/or carboxylated species of osteocalcin (OC), uncarboxylated and/or carboxylated species of matrix Gla-protein (MGP), and uncarboxylated and/or carboxylated species of Gla-rich protein (GRP)

12. A method according to claim 11, wherein said one or more uncarboxylated and/or carboxylated forms of Gla-proteins are selected from the group consisting of dp-ucMGP, ucOC and ucGRP.

13. Use of a conformation-specific assay for either OC, MGP or GRP in the follow-up of patients after organ transplantation.

14. Use of a pharmaceutical or nutraceutical composition according to any one of claims 1 to 8 or a method according to any one of claims 9 to 12 or the use according to claim 13, wherein said organ transplantation is kidney transplantation and the patients are kidney transplant patients.
